# EUROPEAN PATENT APPLICATION

(11) **EP 4 464 319 A1**
(43) Date of publication of application: **20.11.2024**
(21) Application number: 23755838.2
(22) Date of filing: 16.02.2023
(51) Int. Cl.: A61K 31/427, A61K 31/454, A61K 31/4184, A61K 31/417, A61P 15/00, A61P 25/02, A61P 29/00, A61P 17/04

(54) **PHARMACEUTICAL COMPOSITION HAVING ANALGESIC AND/OR ANTIPRURITIC FUNCTION AND USE THEREOF**

(30) Priority: 17.02.2022 CN 202210147495
(71) Applicant: Pengekiphen Biotech Limited Company, Suzhou, Jiangsu 215347 (CN)
(72) Inventor: LIU, Jianping, Suzhou, Jiangsu 215347 (CN); PENG, Xiaomei, Suzhou, Jiangsu 215347 (CN)
(74) Representative: Vitina, Maruta
(86) International application number: PCT/CN2023/076481
(87) International publication number: WO 2023/155837

(57) **Abstract**

A pharmaceutical composition with analgesic and/or antipruritic functions, including a voltage-gated sodium channel 1.7 (Naᵥ1.7) inhibitor and a voltage-gated sodium channel 1.8 (Naᵥ1.8) inhibitor. A weight ratio of the Naᵥ1.7 inhibitor to the Naᵥ1.8 inhibitor is 1:0.001-5000. The composition of the present invention can remarkably relieve pain, can relieve itching, and can also treat and/or prevent tactile sensitive premature ejaculation or be used for anesthesia.

## Description

### CROSS-REFERENCE TO RELATED APPLICATIONS

This application claims the benefit of priority from Chinese Patent Application No. 202210147495.5, filed on February 17, 2022. The content of the aforementioned application, including any intervening amendments made thereto, is incorporated herein by reference in its entirety.

### TECHNICAL FIELD

This application relates to pharmaceutical chemistry, and more particularly to a pharmaceutical composition with analgesic and/or antipruritic functions and an application thereof.

### BACKGROUND

Treatment of pain and itching has been clinical challenges. Voltage-gated sodium channels (Naᵥs) Naᵥ1.7 and Naᵥ1.8 are highly expressed in somatosensory neurons, and play important roles in the perception of itch and pain, and mechanical sensation. It has been demonstrated that Naᵥ1.7 and Naᵥ1.8 exhibit different expression patterns in the dorsal root ganglia (DRG). In mice, Naᵥ1.7 is expressed in large, medium and small neurons, and is more highly expressed in small and medium neurons, while Naᵥ1.8 is expressed predominantly in small and medium neurons, and is not expressed or expressed at an extremely low level in large neurons. Naᵥ1.7 and Naᵥ1.8 are co-expressed in small- and medium-sized DRG neurons. Loss-of-function mutations in Naᵥ1.7 cause congenital insensitivity to pain (CIP) in humans, and the knockout of Naᵥ1.7 will also lead to the loss of pain perception in mice. These research results indicate that it is possible to reach the analgesic effect by inhibiting the Naᵥ1.7 alone. As a highly potent Naᵥ1.7 inhibitor, PF-05089771 does not exhibit desired analgesic effect in clinical studies (A.McDonnell., et al., Efficacy of the Nav1.7 blocker PF-05089771 in a randomized, placebo-controlled, double-blind clinical study in subjects with painful diabetic peripheral neuropathy. Pain. 2018 Aug; 159(8): 1465-1476.). Although there are various selective small molecule inhibitors of Naᵥ1.7 and Naᵥ1.8, the combined use of Naᵥ1.7 inhibitors and Naᵥ1.8 inhibitors to treat and/or prevent pain in clinic has not been reported yet.

### SUMMARY

An object of the disclosure is to provide a pharmaceutical composition with analgesic and/or antipruritic functions and an application thereof, so as to overcome the technical problem in the prior art that the therapy solely targeting voltage-gated sodium channel 1.7 (Naᵥ1.7) or voltage-gated sodium channel 1.8 (Naᵥ1.8) has poor therapeutic effect.

It has been thoroughly investigated and speculated that the downregulation of micro-ribonucleic acid-96 (*miR-96*) caused by the nerve injury not only leads to the upregulated expression of Naᵥ1.7 and Naᵥ1.8, but also may increase the number of dorsal root ganglia (DRG) neurons that co-express Naᵥ1.7 and Naᵥ1.8. Moreover, the simultaneous inhibition of Naᵥ1.7 and Naᵥ1.8 may achieve better analgesic, antipruritic, and even anesthetic effects. Therefore, a *miR-96* knockout mouse is adopted as an *in vivo* model to screen a composition in which there is a synergistic effect between a Naᵥ1.7 inhibitor and a Na,1.8 inhibitor.

In order to achieve the above object, the following technical solutions are adopted.

In a first aspect, this application provides a pharmaceutical composition with analgesic and/or antipruritic functions, comprising:
a Naᵥ1.7 inhibitor; and
a Naᵥ1.8 inhibitor;
wherein a weight ratio of the Naᵥ1.7 inhibitor to the Naᵥ1.8 inhibitor is 1:0.001-5000; and
the Naᵥ1.7 inhibitor is a compound represented by formula (1) or a pharmaceutically-acceptable salt thereof, and the Naᵥ1.8 inhibitor is a compound represented by formula (3) or a pharmaceutically-acceptable salt thereof;
the Naᵥ1.7 inhibitor is a compound represented by formula (2) or a pharmaceutically-acceptable salt thereof, and the Naᵥ1.8 inhibitor is a compound represented by the formula (3) or a pharmaceutically-acceptable salt thereof; or
the Naᵥ1.7 inhibitor is a compound represented by the formula (2) or a pharmaceutically-acceptable salt thereof, and the Naᵥ1.8 inhibitor is a compound represented by formula (4) or a pharmaceutically-acceptable salt thereof;
wherein the formula (1), the formula (2), the formula (3) and the formula (4) are respectively shown as follows:

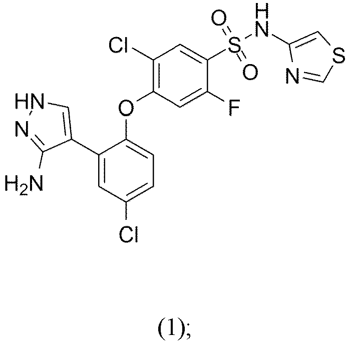

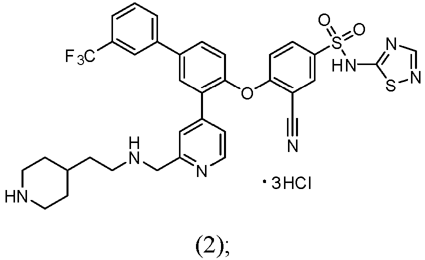

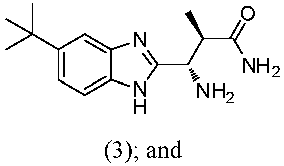

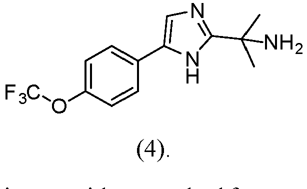

In a second aspect, this application provides a method for treating and/or preventing a disease associated with a voltage-gated sodium channel in a subject in need thereof, comprising: administering to the subject a therapeutically effective amount of the pharmaceutical composition of claim 1.

Compared to the prior art, this application has the following beneficial effects.

The pharmaceutical composition provided by present disclosure can significantly relieve pain and itching, and can be applied in the treatment and/or prevention of premature ejaculation caused by tactile sensitivity or in the anaesthetization.

### BRIEF DESCRIPTION OF THE DRAWINGS

Fig. 1 shows paw withdrawal threshold of microRNA-96 knockout (*miR-96*^{*-*/*-*}) mice in **Example 1** of the present disclosure, where B-F respectively show analgesic effects of intragastric administration of PF-06305591, PF-06456384, PF-05089771, PF-04885614 and CNV1014802 on the *miR-96*^{*-*/*-*} mice (n=8, **P*<0.05, ***P*<0.01, ****P*<0.001, *****P*<0.0001, One-way analysis of variance (ANOVA));
Fig. 2 shows an analgesic effect of intragastric administration of gabapentin on the *miR-96*^{*-* /-} mice in **Example 1** of the present disclosure (n=8, ***P*<0.001, Mann-Whitney test);
Fig. 3 shows an analgesic effect of intragastric administration of a pharmaceutical composition containing PF-06305591 and PF-05089771 on *miR-96*^{*-*/*-*} mice in **Example 2** of the present disclosure (n=8, ***P*<0.01, ****P* <0.001, one-way ANOVA);
Fig. 4 shows an analgesic effect of intragastric administration of a pharmaceutical composition containing PF-06305591 and PF-06456384 on *miR-96*^{*-*/*-*} mice in **Example 3** of the present disclosure (n=8, ***P*<0.01, ****P*<0.001, One-way ANOVA);
Fig. 5 shows an analgesic effect of intragastric administration of a pharmaceutical composition containing PF-06456384 and PF-04885614 on *miR-96*^{*-*/*-*} mice in **Example 4** of the present disclosure (n=8, **P*<0.05, ***P*<0.01, One-way ANOVA);
Fig. 6 shows analgesic effects of cutaneous application of a pharmaceutical composition containing PF-06305591 and PF-05089771 (A), a pharmaceutical composition containing PF-06305591 and PF-06456384 (B) and a pharmaceutical composition containing PF-06456384 and PF-04885614 (C) on *miR-96*^{*-*/*-*} mice in **Example 5** of the present disclosure (n=8, **P*<0.05, ***P*<0.01, One-way ANOVA);
Fig. 7 shows a relieving effect of cutaneous application of a pharmaceutical composition containing PF-06305591 and PF-06456384 on the chloroquine-induced itch in **Example 6** of the present disclosure (n=6, *****P*<0.0001, unpaired t-test);
Fig. 8 shows a relieving effect of cutaneous application of a pharmaceutical composition containing PF-06305591 PF-05089771 or a pharmaceutical composition containing PF-06456384 and PF-04885614 on the chloroquine-induced itch in **Example 6** of the present disclosure (n=6, *****P*<0.0001, unpaired t-test); and
Fig. 9 shows comparison of an analgesic effect of intragastric administration of a pharmaceutical composition containing CNV1014802 and PF-04885614 and that of intragastric administration of CNV1014802 or PF-04885614 alone in **Comparative Example** of the present disclosure (n=8, ****P*<0.001, Mann-Whitney test).

### DETAILED DESCRIPTION OF EMBODIMENTS

Endpoints and any values of ranges disclosed herein are not limited to the exact ranges or values, and these ranges or values should be understood to include values close to these ranges or values. For numerical ranges, endpoint values of each range, endpoint values and individual point values of each range, and the individual point values can be combined with each other to obtain one or more new numerical ranges, and these numerical ranges should be regarded as specifically disclosed herein.

In the present disclosure, a "pharmaceutically-acceptable salt" can be prepared with inorganic acids or organic acids. Salts derived from inorganic acids include hydrochloride, toluenesulfonate, sulfate, hydrobromide, nitrate, phosphate, etc. Salts derived from organic acids include acetate, propionate, glycolate, pyruvate, oxalate, malonate, citrate, maleate, tartrate, pamoate, etc.

The term "pharmaceutically-acceptable amino acid-like compound formed with an amino acid as a carrier" refers to a compound similar to an amino acid formed by reaction of an amine group on the compound with a carboxyl group on the amino acid, such as compounds similar to amino acids formed by reaction with valine, threonine or phenylalanine.

Term "isomer" includes, but is not limited to, stereoisomers, enantiomers, and diastereomers.

The term "solvate" refers to a complex formed by the combination of a compound represented by each of Formulas (1)-(4) or a derivative thereof and a solvent.

An "isotopic variant" includes, but is not limited to, deuterated variants.

The term "hydrate" refers to a compound containing water, where the water molecule can be connected to parts shown in the Formulas (1)-(4) through a coordination bond or a covalent bond.

The term "polymorph" refers to different crystal structures of a crystalline compound. Different polymorphic compounds may be caused by different crystal stacking (stacking polymorphism) or different stacking between different conformers of the same molecule (conformational polymorphism).

The term "prodrug", also known as precursor chemical, drug precursor or precursor, refers to a compound obtained by chemical structural modification of a drug, which is inactive or less active in vitro, and exerts its efficacy in vivo through enzymatic or non-enzymatic conversion to release an active drug. For example, amino groups in the Formulas (1)-(4) can be modified to form amide compounds, amino ester compounds and amidine compounds. In a first aspect, a pharmaceutical composition with analgesic and/or antipruritic functions is provided, including a voltage-gated sodium channel 1.7 (Naᵥ1.7) inhibitor and a voltage-gated sodium channel 1.8 (Naᵥ1.8) inhibitor.

A weight ratio of the Naᵥ1.7 inhibitor to the Naᵥ1.8 inhibitor can be 1:0.001-5000, preferably 1:0.005-10, and more preferably 1:0.01-2.

In some embodiments, the Naᵥ1.7 inhibitor can be a compound represented by the Formula (1) (referred to as Compound 1) or a compound represented by the Formula (2) (referred to as Compound 2), or at least one of a pharmaceutically-acceptable salt, a pharmaceutically-acceptable amino acid-like compound formed with an amino acid as carrier, an isomer, a solvate, an isotopic variant, a hydrate, a polymorph, and a prodrug thereof.

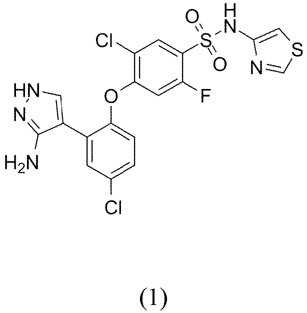

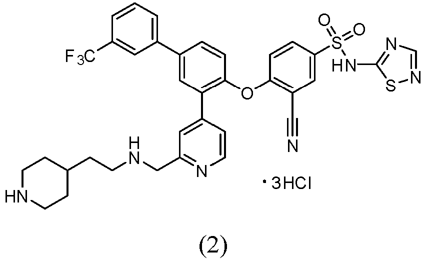

In some embodiments, the Naᵥ1.8 inhibitor is a compound represented by the Formula (3) (referred to as Compound 3) or a compound represented by the Formula (4) (referred to as Compound 4), or at least one of a pharmaceutically-acceptable salt, a pharmaceutically-acceptable amino acid-like compound formed with an amino acid as a carrier, an isomer, a solvate, an isotopic variant, a hydrate, a polymorph, and a prodrug thereof.

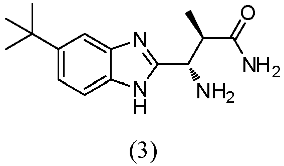

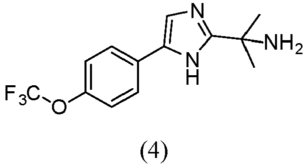

In an embodiment, the Naᵥ1.7 inhibitor is Compound 1 or the pharmaceutically-acceptable salt thereof, and the Naᵥ1.8 inhibitor is Compound 3 or the pharmaceutically-acceptable salt thereof; and/or the weight ratio of the Naᵥ1.7 inhibitor to the Naᵥ1.8 inhibitor is 1:0.005-0.5.

In an embodiment, the Naᵥ1.7 inhibitor is Compound 2 or the pharmaceutically-acceptable salt thereof, the Naᵥ1.8 inhibitor is Compound 3 or the pharmaceutically-acceptable salt thereof; and/or the weight ratio of the Naᵥ1.7 inhibitor to the Naᵥ1.8 inhibitor is 1:0.01-0.1. In an embodiment, the Naᵥ1.7 inhibitor is Compound 2 or the pharmaceutically-acceptable salt thereof, the Naᵥ1.8 inhibitor is Compound 4 or the pharmaceutically-acceptable salt thereof; and/or the weight ratio of the Naᵥ1.7 inhibitor to the Naᵥ1.8 inhibitor is 1 :0.05-0.2. In some specific embodiments of the present disclosure, in addition to the above combinations of the Naᵥ1.7 inhibitor and the Naᵥ1.8 inhibitor, Compounds 1-4 can also be used in combination with other Naᵥ1.7 inhibitors or other Naᵥ1.8 inhibitors with inhibitory activity to form an effective combination regimen that can be obtained by those skilled in the art.

In an embodiment, the pharmaceutical composition further includes a pharmaceutically-acceptable carrier.

In an embodiment, the pharmaceutically-acceptable carrier is selected form the group consisting of a solvent, an excipient, a dispersion medium, a coating, a transdermal agent, an isotonic agent, an absorption-delaying agent and a combination thereof.

In an embodiment, the pharmaceutically-acceptable carrier is selected form the group consisting of starch, microcrystalline cellulose, lactose, sucrose, mannitol, toluene sulfonate, hydrochloride, hydroxypropyl cellulose, sodium carboxymethyl starch, cross-linked polyvinyl pyrrolidone, sodium alginate, agar, hydroxypropyl methylcellulose, methylcellulose, hydroxyethyl cellulose, Carbopol (crosslinked polyacrylic acid polymer), polyvinyl alcohol, acrylic resin, chitosan, beeswax, stearic acid and a combination thereof. In an embodiment, a total weight of the Naᵥ1.7 inhibitor and the Naᵥ1.8 inhibitor is 0.0001-99.9999% of a weight of the pharmaceutical composition.

In a second aspect, an application of a pharmaceutical composition in the preparation of a drug for treating and/or preventing a disease associated with a voltage-gated sodium ion channel is provided.

In an embodiment, the voltage-gated sodium ion channel can be Naᵥ1.7, Naᵥ1.8 or a combination thereof.

In an embodiment, the disease can be a pain and/or an itch, or a premature ejaculation caused by tactile sensitivity.

In an embodiment, the pain is selected form the group consisting of a traumatic pain, an intraoperative pain, a postoperative pain, an inflammatory pain, a neuropathic pain, a headache, a cervical spondylosis, a shoulder pain, a low back pain, a toothache, a chest pain, an abdominal pain, a lower limb pain, a muscle and bone pain, a fibromyalgia, a glossopharyngeal neuralgia, a trigeminal neuralgia, a sciatica, a multiple sclerosis-related neuralgia, a diabetic neuralgia, a cancer-related pain, a postherpetic neuralgia, a human immunodeficiency virus (HIV)-related neuralgia, a post-burn pain, an arthritis pain, a dysmenorrhea, a visceral pain and a combination thereof.

In the present disclosure, the inflammatory pain refers to a pain caused by stimulation of sensory nerve endings by various mediators produced by tissue damage, such as a prostaglandin, an inflammatory factor, a chemokine and adenosine, and the neuropathic pain refers to a pain caused by damage to a central nervous system or peripheral nervous system or a functional disorder after damage.

The itch refers to skin itching, including itch caused by skin diseases and pruritus caused by only skin itching without primary skin damage. In the present disclosure, the itch can be selected from the group consisting of a histamine-dependent itch, a non-histamine-dependent itch, a chemical itch, a mechanical itch, an insect bite-induced itch, a liver disease-induced itch, a chronic kidney disease-induced itch and a combination thereof.

In addition, the present disclosure also involves a method for treating a disease associated with a voltage-gated sodium ion channel in a subject in need thereof, including the following step. A therapeutically effective amount of the above pharmaceutical composition is administered to the subject. The pharmaceutical composition can be administered in a conventional manner. An administration route of the pharmaceutical composition can include, but is not limited to: oral administration, intra-oral administration, injection (such as intramuscular injection, subcutaneous injection, intrathecal injection, epidural injection and intravenous injection), respiratory administration, cutaneous administration (such as cutaneous application and cutaneous spray), ophthalmic administration, nasal mucosal administration, rectal administration, vaginal administration, otic administration and dialysis. The subject can be an animal, including a mammal (especially a primate, such as a human), a rodent (such as a mouse), livestock, poultry, a pet and an experimental animal. Medical personnel can select a dosage of the pharmaceutical composition according to a physical condition of the subject. For example, for an adult, the dosage can be 0.01-10 mg/kg or 0.01-1 mg/cm².

According to the present disclosure, the term "treatment" refers to a therapeutic as well as a prophylactic or inhibitory measure containing any clinically desired or beneficial effect for a disease or condition (including, but not limited to, relief or alleviation of one or more symptoms, and regression, slowing, or cessation of the progression of a disease or condition). The embodiments of the present disclosure will be described in detail below. All the following animal experiments were performed in compliance with an ethical approval published by the Laboratory Animal Ethics Committee of Suzhou Institute of Biomedical Engineering and Technology, Chinese Academy of Sciences.

### EXAMPLE 1

The efficacy of an individual drug compound was investigated herein.

In order to screen an analgesic drug with excellent oral efficacy, analgesic effects of intragastric administration of Naᵥ1.7 inhibitor and Naᵥ1.8 inhibitor in eight 3-6-month-old *miR-96*^{*-*/*-*} mice (Kunshan Pengji Kaifeng Biotechnology Co., Ltd.) were tested.

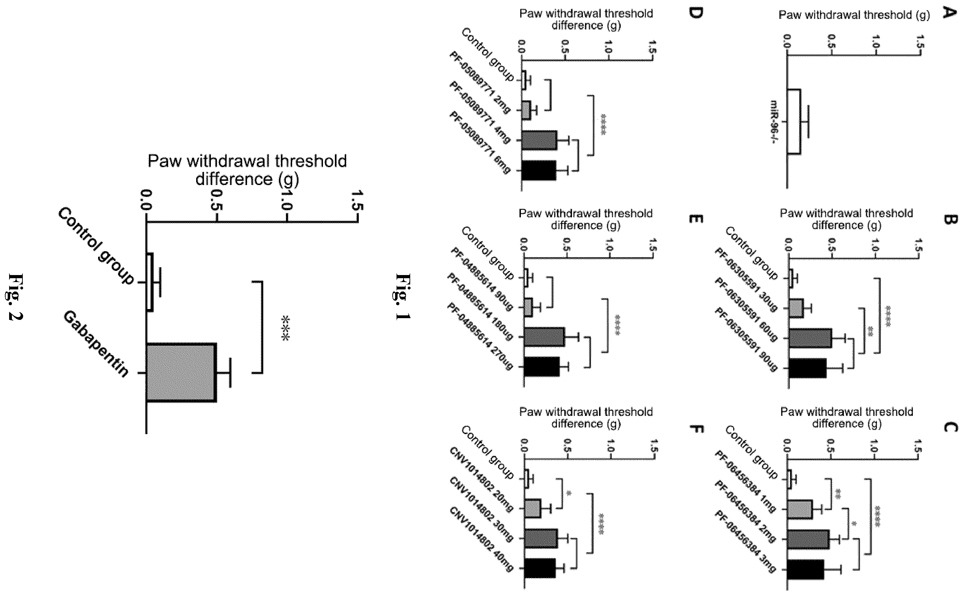

Firstly, mechanical pain thresholds of hind paws of the *miR-96*^{*-*/*-*} mice were measured. The eight *miR-96*^{*-*/*-*} mice were placed on an iron frame and covered with a plexiglass cover. After 30 min, paw withdrawal thresholds (PWT) of the mice were measured using Von Frey (DanMic Global, CA, USA). An average threshold of the mice was 0.16 g±0.07 g, as shown in "A" of Fig. 1, belonging to the mechanical allodynia. PF-06305591 (Compound 3, (αR, βS)-β-Amino-6-(1,1-dimethylethyl)-α-methyl-1H-benzimidazole-2-propanamide dihydrate, Sigma, PZ0297-25MG, a half maximal inhibitory concentration (IC₅₀) for hNaᵥ1.8=15 nM) was dissolved with a 10% dimethylsulfoxide (DMSO)/normal saline solution (V/V). 1 h after the intragastric administration of 30 µg/kg, 60 µg/kg or 90 µg/kg of PF-06305591, the mechanical pain thresholds of the hind paws of the *miR-96*^{*-*/*-*} mice were measured. The test result showed that analgesic effects of the 60 µg/kg-dose group and the 90 µg/kg-dose group were significantly better than that of the solvent control group, and the 60 µg/kg-dose group exhibited the best effect, as shown in "B" of Fig. 1.

PF-06456384 (Compound 2, 3-cyano-4-[[3-[2-[[[2-(4-piperidinyl)ethyl]amino]methyl]-4-pyridinyl]-3'-(trifluoromethyl)[1,1'-biphenyl]-4-yl]oxy]-N-1,2,4-thiadiazol-5-yl-benzenesulfonamide trihydrochloride, Sigma, PZ0386-25MG, IC₅₀ for hNaᵥ1.7=0.01 nM) was dissolved with a 10% DMSO/normal saline solution (V/V). 1 h after intragastric administration of 1 mg/kg, 2 mg/kg or 3 mg/kg of PF-06456384, the mechanical pain thresholds of hind paws of the *miR-96*^{*-*/*-*} mice were measured. The test result showed that compared to the solvent control group, the three dose groups all exhibited significant analgesic effects, and the 2 mg/kg-dose group had the best efficacy, as shown in "C" of Fig. 1.

PF-05089771 (Compound 1, 4-[2-(3-amino-1H-pyrazol-4-yl)-4-chlorophenoxy]-5-chloro-2-fluoro-N-4-thiazolylbenzenesulfonamide tosylate, IC₅₀ for hNaᵥ1.7=11 nM) was dissolved with a 30% DMSO/normal saline solution (V/V). 1 h after intragastric administration of 2 mg/kg, 4 mg/kg or 6 mg/kg of PF-05089771, the mechanical pain thresholds of hind paws of the *miR-96*^{*-*/*-*} mice were measured. The test result showed that analgesic effects of the 4 mg/kg-dose group and the 6 mg/kg-dose group were significantly better than that of the solvent control group, and the efficacy of the 6 mg/kg-dose group was not better than that of the 4 mg/kg-dose group, as shown in "D" of Fig. 1.

PF-04885614 (Compound 4, 1-methyl-1-[4-(4-trifluoromethoxy-phenyl)-1H-imidazol-2-yl]-ethylamine, IC₅₀ for hNaᵥ1.8=53 nM) was dissolved with a 10% DMSO/normal saline solution (V/V). 1 h after intragastric administration of 90 µg/kg, 180 µg/kg or 270 µg/kg of PF-04885614, the mechanical pain thresholds of hind paws of the *miR-96*^{*-*/*-*} mice were measured. The test result showed that analgesic effects of the 180 µg/kg-dose group and the 270 µg/kg-dose group were significantly better than that of the solvent control group, and the 180 µg/kg-dose group exhibited the best effect, as shown in "E" of Fig. 1.

CNV1014802 (IC₅₀ for hNaᵥ1.7=32 µM) was dissolved with a 30% DMSO/normal saline solution (V/V). 1 h after intragastric administration of 20 mg/kg, 30 mg/kg or 40 mg/kg of CNV1014802, the mechanical pain thresholds of hind paws of the *miR-96*^{*-*/*-*} mice were measured. It was found that compared to the solvent control group, the three dose groups all exhibited significant analgesic effects, and the 30 mg/kg-dose group had the best effect, as shown in "F" of Fig. 1.

Gabapentin was dissolved with normal saline. 1 h after intragastric administration of 50 mg/kg of gabapentin, the mechanical pain thresholds of hind paws of the *miR-96*^{*-*/*-*} mice were measured. The test result showed that 50 mg/kg of gabapentin can significantly improve the mechanical pain thresholds of the mice (the paw withdrawal threshold difference was 0.5 g±0.09 g), as shown in Fig. 2.

### EXAMPLE 2

The efficacy of a pharmaceutical composition was investigated.

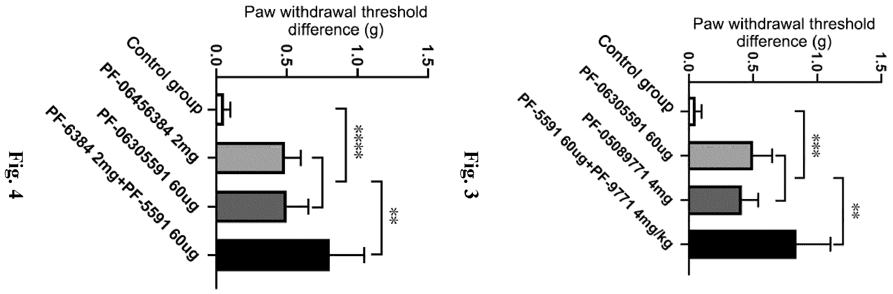

A pharmaceutical composition including PF-06305591 and PF-05089771 was dissolved with a 30% DMSO/normal saline solution (V/V). 1 h after intragastric administration of the pharmaceutical composition (dose of the PF-06305591: 60 µg/kg; and dose of the PF-05089771: 4 mg/kg), mechanical pain thresholds of hind paws of the *miR-96*^{*-*/*-*} mice were measured. The test result showed that a combination of 60 µg/kg of PF-06305591 and 4 mg/kg of PF-05089771 can significantly alleviate the mechanical pain of the *miR-96*^{*-*/*-*} mice, which was significantly superior to the alone use of 60 µg/kg of PF-06305591 or 4 mg/kg of PF-05089771 in terms of therapeutic effect, as shown in Fig. 3 (where "PF-5591" represented "PF-06305591", and "PF-9771" represented "PF-05089771").

### EXAMPLE 3

The efficacy of a pharmaceutical composition was investigated herein.

A pharmaceutical composition including PF-06305591 and PF-06456384 was dissolved with a 10% DMSO/normal saline solution (V/V). 1 h after intragastric administration of the pharmaceutical composition (dose of the PF-06305591: 60 µg/kg; and dose of the PF-06456384: 2 mg/kg), mechanical pain thresholds of hind paws of *miR-96*^{*-*/*-*} mice were measured. The test result showed that a combination of 60 µg/kg of PF-06305591 and 2 mg/kg of PF-06456384 can significantly alleviate the mechanical pain of the *miR-96*^{*-*/*-*} mice, which was significantly superior to the alone use of 60 µg/kg of PF-06305591 or 4 mg/kg of PF-05089771 in terms of therapeutic effect, as shown in Fig. 4 (where "PF-5591" represented "PF-06305591", and "PF-6384" represented "PF-06456384").

### EXAMPLE 4

The efficacy of a pharmaceutical composition was investigated herein.

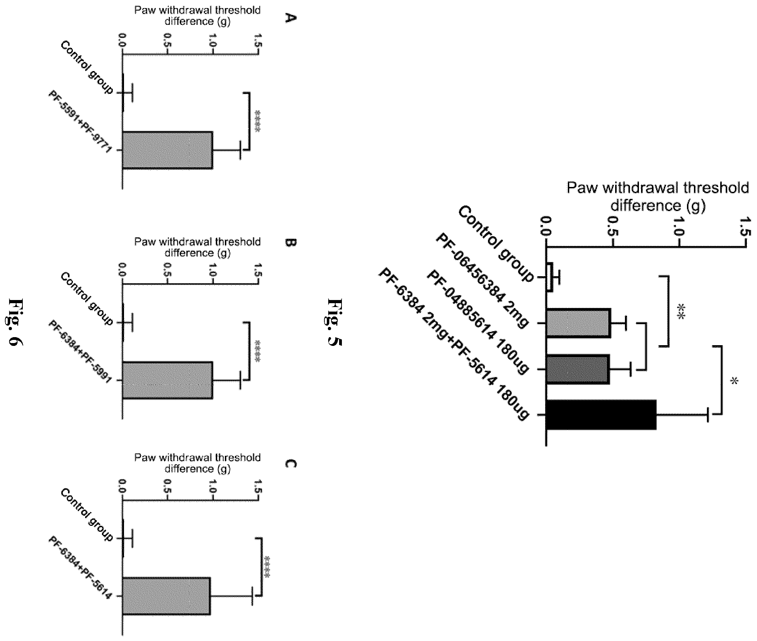

A pharmaceutical composition including PF-06456384 and PF-04885614 was dissolved with a 10% DMSO/normal saline solution (V/V). 1 h after intragastric administration of the pharmaceutical composition (dose of the PF-06456384: 2 mg/kg; and dose of the PF-04885614: 180 µg/kg), mechanical pain thresholds of hind paws of *miR-96*^{*-*/*-*} mice were measured. The test result showed that a combination of 2 mg/kg of PF-06456384 and 180 µg/kg of PF-04885614 can significantly alleviate the mechanical pain of the *miR-96*^{*-*/*-*} mice, which was significantly superior to the alone use of 180 µg/kg of PF-04885614 or 2 mg/kg of PF-06456384 in terms of therapeutic effect, as shown in Fig. 5 (where "PF-5614" represented "PF-04885614" and "PF-6384" represented "PF-06456384").

### EXAMPLE 5

The analgesic effect of cutaneous application was investigated herein.

In order to test the analgesic effect of cutaneous application of a combination of a Naᵥ1.7 inhibitor and a Naᵥ1.8 inhibitor, *miR-96*^{*-*/*-*} mice were anesthetized with isoflurane, and 10 µL of a 30% DMSO/normal saline solution (V/V) containing 1 mg/mL PF-06305591 and 20 mg/mL PF-05089771 was spread on a left limb of each of the *miR-96*^{*-*/*-*} mice. 5 min later, the mice were transferred from the anesthesia machine to an iron frame under a plexiglass cover. 30 min after the administration, the mechanical pain thresholds of the mice were measured.

The test result showed that the application of the combination of PF-06305591 and PF-05089771 can significantly improve the mechanical pain thresholds of the *miR-96*^{*-*/*-*} mice, as shown in "A" of Fig. 6 (where "PF-5591" represented "PF-06305591", and "PF-9771" represented "PF-05089771").

Similarly, 10 µL of a 10% DMSO/normal saline solution (V/V) containing 1 mg/mL PF-06305591 and 10 mg/mL PF-06456384 was applied, and the mechanical pain thresholds were measured 30 min later. The results showed that the mechanical pain thresholds of the *miR-96*^{*-*/*-*} mice were significantly improved, as shown in "B" of Fig. 6 (where "PF-6384" represented "PF-06456384", and "PF-5591" represented "PF-06305591").

Similarly, 30 min after spreading 10 µL of a 10% DMSO/normal saline solution (V/V) containing 10 mg/mL PF-06456384 and 2 mg/mL PF-04885614 was applied, and the mechanical pain thresholds were measured 30 min later. The results showed that the mechanical pain thresholds of the *miR-96*^{*-*/*-*} mice were significantly improved, as shown in "C" of Fig. 6 (where "PF-6384" represented "PF-06456384", and "PF-5614" represented "PF-04885614").

### EXAMPLE 6

The antipruritic effect of cutaneous application was investigated herein.

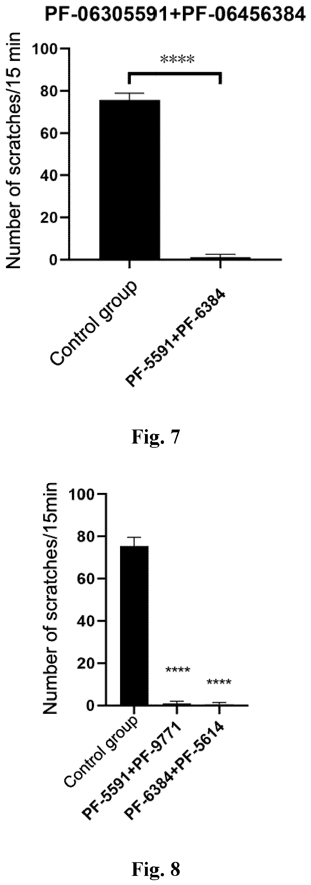

In order to test an antipruritic effect of cutaneous application of a Naᵥ1.7 inhibitor and a Naᵥ1.8 inhibitor, six 8-week-old C57BL/6 mice were anesthetized with isoflurane. The hair behind the ears and at the neck of the mice was removed to form an application area, to which 50 µL of a 10% DMSO/normal saline solution (V/V) containing 1 mg/mL PF-06305591 and 10 mg/mL PF-06456384 was applied. 5 min later, the mice were removed from the anesthesia machine, and subcutaneously injected with 50 µL of 12 mM chloroquine (Sigma Aldrich, CAS 50-63-5) at the application area. Videos were recorded, and the number of scratches of the mice was counted. The statistical analysis results showed that the application of the combination of PF-06305591 and PF-06456384 can significantly inhibit the itch induced by chloroquine, as shown in Fig. 7 (where "PF-6384" represented "PF-06456384", and "PF-5591" represented "PF-06305591").

Similarly, six 8-week-old C57/B16 mice were anesthetized with isoflurane. The hair behind the ears and at the neck of the mice was removed to form an application area, to which 50 µL of a 30% DMSO/normal saline solution (V/V) containing 1 mg/mL PF-06305591 and 20 mg/mL PF-05089771 was applied. 5 min later, the mice were removed from the anesthesia machine, and subcutaneously injected with 50 µL of 12 mM chloroquine at the application area. Videos were recorded, and the number of scratches of the mice was counted. The statistical analysis results showed that the application of the combination of PF-06305591 and PF-05089771 can significantly inhibit the itch induced by chloroquine, as shown in Fig. 8 (where "PF-5591" represented "PF-06305591", and "PF-9771" represented "PF-05089771").

Similarly, 50 µL of a 10% DMSO/normal saline solution (V/V) containing 10 mg/mL PF-06456384 and 2 mg/mL PF-04885614 was applied. 5 min later, the mice were removed from the anesthesia machine, and subcutaneously injected with 50 µL of 12 mM chloroquine at the spreading area. The number of scratches of the mice was counted. The statistical analysis results showed that the application of the combination of PF-06456384 and PF-04885614 can significantly inhibit the itch induced by chloroquine, as shown in Fig. 8 (where "PF-6384" represented "PF-06456384", and "PF-5614" represented "PF-04885614").

### COMPARATIVE EXAMPLE

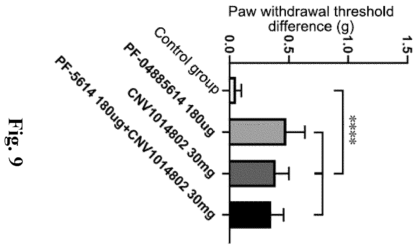

The efficacy of a combination of CNV1014802 and PF-04885614 was investigated herein. The combination ofCNV1014802 and PF-04885614 was dissolved in a 30% DMSO/normal saline solution (V/V). 1 h after intragastric administration of the pharmaceutical composition (dose of the CNV1014802: 30 mg/kg; and dose of the PF-04885614: 180 µg/kg), mechanical pain thresholds of hind paws of *miR-96*^{*-*/*-*} mice were measured. The test result showed that the combination of 30 mg/kg CNV1014802 and 180 µg/kg PF-04885614 on was not superior to the alone use of 30 mg/kg CNV1014802 or 180 µg/kg PF-04885614 in terms of the effect on alleviating the mechanical pain of the *miR-96*^{*-*/*-*} mice, as shown in Fig. 9 (where "PF-5614" represented "PF-04885614").

In summary, the present disclosure can significantly relieve pain by intragastric administration or skin spreading of the pharmaceutical composition containing PF-06305591 and PF-05089771, or the pharmaceutical composition containing PF-06305591 and PF-06456384, or the pharmaceutical composition containing PF-06456384 and PF-04885614. In addition, skin spreading of the pharmaceutical composition containing PF-06305591 and PF-06456384, or the pharmaceutical composition containing PF-06305591 and PF-05089771, or the pharmaceutical composition containing PF-06456384 and PF-04885614 can also relieve itching. However, in the Comparative Example, the combination of the Naᵥ1.7 inhibitor CNV1014802 and the Naᵥ1.8 inhibitor PF-04885614 fails to exhibit an analgesic effect superior to that of a single inhibitor. It can be concluded that the specific composition of the Naᵥ1.7 inhibitor and the Naᵥ1.8 inhibitor provided by the present disclosure can prevent and/or treat diseases associated with Naᵥ1.7 and Naᵥ1.8 activity, including but not limited to pain and itch.

## Claims

1. A pharmaceutical composition with analgesic and/or antipruritic functions, comprising:
a voltage-gated sodium channel 1.7 (Naᵥ1.7) inhibitor; and
a voltage-gated sodium channel 1.8 (Naᵥ1.8) inhibitor;
**characterized in that** a weight ratio of the Naᵥ1.7 inhibitor to the Naᵥ1.8 inhibitor is 1:0.001-5000; and
the Naᵥ1.7 inhibitor is a compound represented by formula (1) or a pharmaceutically-acceptable salt thereof, and the Naᵥ1.8 inhibitor is a compound represented by formula (3) or a pharmaceutically-acceptable salt thereof;
the Naᵥ1.7 inhibitor is a compound represented by formula (2) or a pharmaceutically-acceptable salt thereof, and the Naᵥ1.8 inhibitor is a compound represented by the formula (3) or a pharmaceutically-acceptable salt thereof; or
the Naᵥ1.7 inhibitor is a compound represented by the formula (2) or a pharmaceutically-acceptable salt thereof, and the Naᵥ1. 8 inhibitor is a compound represented by formula (4) or a pharmaceutically-acceptable salt thereof;
wherein the formula (1), the formula (2), the formula (3) and the formula (4) are respectively shown as follows:

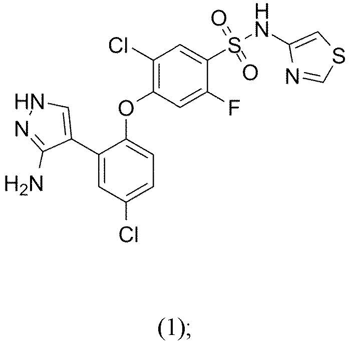

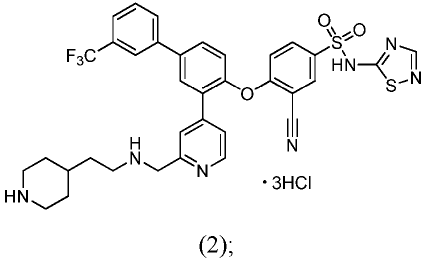

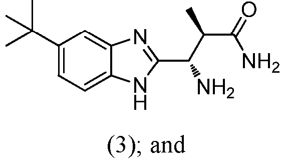

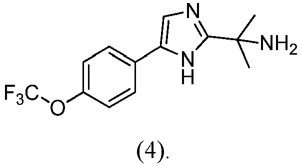

2. The pharmaceutical composition according to claim 1, **characterized in that** the weight ratio of the Naᵥ1.7 inhibitor to the Naᵥ1.8 inhibitor is 1 :0.001-50.

3. The pharmaceutical composition according to claim 1, **characterized in that** the weight ratio of the Naᵥ1.7 inhibitor to the Naᵥ1.8 inhibitor is 1:0.005-10.

4. The pharmaceutical composition according to claim 1, **characterized in that** the weight ratio of the Naᵥ1.7 inhibitor to the Naᵥ1.8 inhibitor is 1 :0.01-2.

5. The pharmaceutical composition according to claim 1 or 2, further comprising:
a pharmaceutically-acceptable carrier.

6. The pharmaceutical composition according to claim 5, **characterized in that** the pharmaceutically-acceptable carrier is selected form the group consisting of a solvent, an excipient, a dispersion medium, a coating, a transdermal agent, an isotonic agent, an absorption-delaying agent and a combination thereof.

7. The pharmaceutical composition according to claim 5, **characterized in that** the pharmaceutically-acceptable carrier is selected form the group consisting of starch, microcrystalline cellulose, lactose, sucrose, mannitol, toluene sulfonate, hydrochloride, hydroxypropyl cellulose, sodium carboxymethyl starch, cross-linked polyvinyl pyrrolidone, sodium alginate, agar, hydroxypropyl methylcellulose, methylcellulose, hydroxyethyl cellulose, crosslinked polyacrylic acid polymer, polyvinyl alcohol, acrylic resin, chitosan, beeswax, stearic acid and a combination thereof.

8. The pharmaceutical composition according to claim 5, **characterized in that** a total weight of the Naᵥ1.7 inhibitor and the Naᵥ1.8 inhibitor is 0.0001-99.9999% of a weight of the pharmaceutical composition.

9. The pharmaceutical composition according to any one of claims 1-8 for use in treatment and/or prevention of a disease associated with a voltage-gated sodium channel.

10. The pharmaceutical composition for use according to claim 9, **characterized in that** the voltage-gated sodium channel is Naᵥ1.7, Naᵥ1.8 or a combination thereof.

11. The pharmaceutical composition for use according to claim 9 or 10, **characterized in that** the disease is a pain, an itch or a combination thereof.

12. The pharmaceutical composition for use according to claim 11, **characterized in that** the pain is selected form the group consisting of a traumatic pain, an intraoperative pain, a postoperative pain, an inflammatory pain, a neuropathic pain, a headache, a cervical spondylosis, a shoulder pain, a low back pain, a toothache, a chest pain, an abdominal pain, a lower limb pain, a muscle and bone pain, a fibromyalgia, a glossopharyngeal neuralgia, a trigeminal neuralgia, a sciatica, a multiple sclerosis-related neuralgia, a diabetic neuralgia, a cancer-related pain, a postherpetic neuralgia, a human immunodeficiency virus (HIV)-related neuralgia, a post-burn pain, an arthritis pain, a dysmenorrhea, a visceral pain and a combination thereof.

13. The pharmaceutical composition for use according to claim 11, **characterized in that** the itch is selected form the group consisting of a histamine-dependent itch, a non-histamine-dependent itch, a chemical itch, a mechanical itch, an insect bite-induced itch, a liver disease-induced itch, a chronic kidney disease-induced itch and a combination thereof.

14. The pharmaceutical composition for use according to claim 9 or 10, **characterized in that** an administration route of the pharmaceutical composition is selected from the group consisting of oral administration, intra-oral administration, intramuscular injection, subcutaneous injection, inhalation administration, cutaneous application, cutaneous spray, ophthalmic administration, nasal mucosal administration, rectal administration, vaginal administration, otic administration, dialysis and a combination thereof.
